# EUROPEAN PATENT SPECIFICATION

(11) **EP 1 112 066 B1**
(45) Date of publication and mention of the grant of the patent: **16.07.2003**
(21) Application number: 99944533.1
(22) Date of filing: 27.08.1999
(51) Int. Cl.: A61K 31/704, A61P 35/00

(54) **USE OF METHOXYMORPHOLINO DOXORUBICIN FOR THE TREATMENT OF A LIVER TUMOR**
VERWENDUNG VON METHOXYMORPHOLINO DOXORUBICIN ZUR BEHANDLUNG EINES LEBERTUMOR
UTILISATION D'UN DERIVE D'ANTHRACYCLINE DANS LE TRAITEMENT D'UNE TUMEUR DU FOIE

(30) Priority: 14.09.1998 GB 9820012
(43) Date of publication of application: 04.07.2001
(73) Proprietor: Pharmacia Italia S.p.A., 20152 Milano (IT)
(72) Inventor: PACCIARINI, Maria, Adele, I-20158 Milano (IT); VALOTA, Olga, I-20025 Legnano (IT); KERR, David, Birmingham B38 8TL (GB)
(86) International application number: EP9906298
(87) International publication number: WO00015203

(56) References cited:
- QUINTIERI, L. ET AL: "Delivery of methoxymorpholinyl doxorubicn by interleukin 2-activated NK cells: Effect in mice bearing hepatic metastases" BRITISH JOURNAL OF CANCER, vol. 79, no. 7-8, March 1999 (1999-03), pages 1067-1073, XP000893191
- VASEY, P.A. ET AL: "Phase I clinical and pharmacokinetic study of 3'-deamino-3'-(2-methoxy-4-morpholinyl)dox orubicin (FCE 23762)" CANCER RESEARCH, vol. 55, no. 10, 1995, pages 2090-2096, XP000891501
- TAUB, R.N. ET AL: "Phase II study of methoxymorpholinodoxorubicin in advanced sarcomas (PNU/S2243 (FCE 23762))" PROCEEDINGS OF THE ANNUAL MEETING OF THE AMERICAN SOCIETY OF CLINICAL ONCOLOGY, vol. 16, 1997, page A1831 XP002135896
- BAKKER, M. ET AL: "Broad phase II and pharmacokinetic study of methoxy-morpholino doxorubicin (FCE 23762-MMRDX) in non-small-cell lung cancer, renal cancer and other solid tumour patients" BRITISH JOURNAL OF CANCER, vol. 77, no. 1, January 1998 (1998-01), pages 139-146, XP000893190
- ROBERT, J. ET AL: "Pharmacokinetics and metabolism of anthracyclines" CANCER SURVEYS, vol. 17, 1993, pages 219-252, XP000893095
- ROUGIER, P. ET AL: "Intérêt des chimiothérapies loco-régionales par les anthracyclines" PATHOLOGIE BIOLOGIE, vol. 35, no. 1, 1987, pages 123-128, XP000893118
- KIRK, S. ET AL: "Irresectable hepatoma treated by intrahepatic iodized oil doxorubicin hydrochloride: Initial results" SURGERY, vol. 109, no. 6, 1991, pages 694-697, XP000893192
- CARR, B.I. ET AL: "Prolonged survival with chemotherapy alone for hepatocellular carcinoma (HCC) with intra-arterial chemotherapy" PROCEEDINGS OF THE AMERICAN SOCIETY OF CLINICAL ONCOLOGY, vol. 13, March 1994 (1994-03), page 205 XP000893111
- CARR, B.I. ET AL: "Phase II study of Spherex (degradable starch microspheres) injected into the hepatic artery in conjunction with doxorubicin and cisplatin in the treatment of advanced-stage hepaocellular carcinoma: Interim analysis" SEMINARS IN ONCOLGY, vol. 24, no. 2 suppl. 6, April 1997 (1997-04), pages S6-97-S6-99, XP000893102

## Description

The present invention relates to the use of methoxymorpholino doxorubicin for the manufacture of a medicament for intrahepatic administration for the treatment of a liver cancer.

Methoxymorpholino doxorubicin (MMDX, internal code PNU 152243) of formula 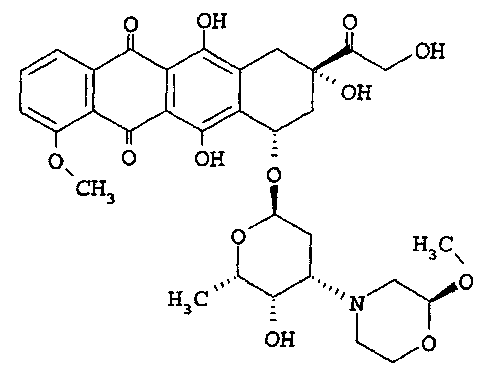 is a new doxorubicin derivative obtained with the substitution of the -NH₂ at position 3' in the sugar moiety with a methoxymorpholino group. The compound was synthesized in the course of a research program aimed at identifying new anthracyclines with at least partially novel modes of action, and possessing broad spectrum of activity, including activity on multidrug resistant (mdr) tumors.

Robert J. et al., Cancer Surveys, Vol. 13, 1993, pages 219-252, describe the metabolic pathway of known anthracyclines such as doxorubicin, epirubicin and idarubicin and the rationale for intra-arterial administration of said anthracyclines.

Kirk S. et al.,Surgery, Vol. 109, No. 6, 1991, pages 694-697 describe the investigation and treatment of 14 patients with primary hepatocellular carcinoma with infusion of iodized oil and doxorubicin hydrochloride.

Vasey et al., Cancer research, Vol. 55, No. 10, 1995, pages 2090-2096, describe phase I clinical and pharmacokinetic studies with MMDX administered by i.v. bolus injection in patients with refractory solid tumors including patients with liver metastases from colorectal cancer.

MMDX is active *in vitro* and *in vivo* on tumor cells resistant to anthracyclines and presenting the mdr phenotype, this last mechanism being recognized to occur also in man.

No cross-resistance was observed on tumor cells resistant to L-PAM or cDDP, or on cells resistant to Topoisomerase II inhibitors (at-mdr).

MMDX is active after i.p., i.v. or oral administration, with good antitumor activity on murine leukemias, and on solid murine and human tumor models.

The compound differs from most anthracyclines in being highly potent when administered *in vivo,* the optimal i.v. dose being at least 80 fold less than that of doxorubicin. This result, and the observation that the cytotoxic activity of MMDX is increased *in vitro* in the presence of mouse, rat and human liver microsomes, suggest that MMDX may be transformed into highly cytotoxic metabolite(s).

A well known pathway of the metabolic transformation of the antitumor anthracyclines in mammals is the side-chain carbonyl group reduction, giving the corresponding 13-dihydro derivative. The reduced derivative of MMDX maintains activity *in vitro* and *in vivo* against doxorubicin-resistant models, at doses however 10 fold higher as compared to the parent drug.

The high lipophilicity of the molecule, which confers to the compound the ability to reach high intracellular concentrations and is most likely one of the reasons of its efficacy on resistant models, makes it effective also after oral administration. The oral antitumor efficacy of MMDX has been examined in a panel of different tumor types with various schedules of administration. The results demonstrate that the oral treatment with MMDX is associated, in all the animal models examined, with an antitumor activity comparable to that observed after intravenous (i.v.) administration. In these models, the effective oral doses of MMDX are 1.3-2 fold higher than the effective i.v. doses. In particular, in liver metastases from M5076 murine fibrosarcoma, the best result (doubling of survival time) was achieved with the oral formulation, administered daily for 5 days; the injectable formulation was less effective. This might be a reflection of a different behaviour of the drug, due to first pass effect to the liver.

It is well know that there is currently no effective conventional treatment for patient with primary hepatocellular carcinoma (HCC) and cholangiocarcinoma invading the liver.

In addition, the liver is a common site of metastasis in many human cancers.

### Primary Liver Cancer

Tumors of the liver are among the most common malignancies in the world. The annual international incidence of the disease is approximately 1 million cases, with a male to female ratio of approximately 4:1. There are 1.2 million deaths per year world wide. There is a huge geographic variation incidence corresponding to 2/100,000 in North America to 30/100,000 in South East Asia, although these numbers refer often to "total liver cancer", without a differentiation between primary and secondary.

The highest incidence of liver cancer is seen in the Far East and is associated with high endemic hepatitis B carrier rates, contamination of foodstuffs, stored grains, drinking water and soil. Advances in the management of these malignancies will likely depend on immunization strategies for hepatitis B and C and on developing a means of decreasing cirrhosis of any origin. Cirrhosis is frequently associated with HCC, especially in Europe and USA. Systemic chemotherapy is generally disappointing, with response rate averaging less than 20%. Anthracyclines remain the most widely used agents. Mitomycin C is also used.

A wide variety of both surgical and nonsurgical therapies have become available for HCC. Surgical resection and orthotopic transplantation are the only curative options, but it is estimated that less than 10% of patients are suitable for this approach and long-term results are poor. The low resectability and the high recurrent rate (40% in five years after surgery), together with the fact that HCC tends to be fatal because of local hepatic progression rather than widespread metastasis, stimulated the development of several locoregional therapeutic approaches, including intra-arterial chemotherapy. Higher response rates appear to be reported for intra hepatic artery (IHA) chemotherapy administered along with embolizing agents, such as LIPIODOL™, gel foam and degradable starch microspheres. This approach is increasingly used in the Far East. Anthracyclines (doxorubicin and epirubicin) are widely used in this setting. However, no substantial improvement in survival is obtained with current chemotherapeutic attempts. The need for new effective treatments remains high.

### Secondary Liver Cancer

Liver is a common site of metastasis in many human cancers and hepatic involvement is often the major cause of morbidity and mortality in disseminated malignancy. In particular, the liver, by virtue of the portal venous drainage system, is usually the first - and may be the only - site of metastases in many patients with primary colorectal cancer. Gastric and pancreatic cancers - but also melanoma, lung and breast cancers - may also frequently metastasize to the liver. Metastatic liver tumors are often the first evidence of the progression of a patient's cancer, and particularly in colorectal cancer are the only tumors detected. Colorectal carcinoma is a disease of industrialized nations. It is estimated that in USA over 160,000 new cases were diagnosed yearly and that 75,000 deaths occurred as a result of the advanced disease. Epidemiological studies show that the incidence of colorectal carcinoma is increasing. Involvement of the liver is found in 40-70% of patients with progressive disease and liver is the sole site of initial tumor recurrence in up to 30% of patients with metastatic disease. Left untreated, metastatic lesions to the liver from colorectal cancers are associated with survival of 3 to 24 months.

For patients with isolated liver metastases, surgical resection is the best treatment option, with 20-30% 5 year survival rate. Surgery is only possible in about 10% of cases and it is extimated that up to 25% of patients undergoing surgical resection will recur with metastatic liver cancer.

Palliation with systemic chemotherapy is currently offered to most patients with extensive or multiple liver metastases. To date, systemic 5-fluoruracil (5-FU) plus folinic acid is considered the optimum treatment for metastatic colorectal cancer, yielding response rates of only 20% and overall survival of around 12 months. Irinotecan hydrochloride trihydrate is the standard treatment after failure of 5-FU leucovorin, with a response rate of 15% and median survival time of approximately 9 months. Clinical trials are ongoing with the objective to determine the role of irinotecan as first-line treatment in combination with 5-FU leucovorin.

In case the disease is confined to the liver and it is inoperable, regional intraarterial chemotherapy may be indicated. With the hepatic arterial infusion of 5-FU or of its analogue, 5-fluorodeoxyuridine (FUDR), attempts have been made to maximise the clinical outcome (response rate in up to 50% of cases) but with no substantial effect on survival.

MMDX represents a therapeutic option in the treatment of a liver cancer.

The expectation that MMDX is effective in liver neoplasms comes from the findings of phase I and phase Ib studies conducted by intravenous route, where, out of 30 patients evaluable for response in liver, 5 experienced regressions of liver metastases. Two patients with colorectal cancer had a <50% regression of liver lesions after 3 cycles, two patients with renal cancer showed a regression greater than 50% in liver lesions after 3 cycles of treatment and an additional patient with colorectal cancer and multiple liver metastases at entry (subsequently dead from pulmonary embolism after the first cycle of treatment) showed no evidence of liver metastases at autopsy. Tumor shrinkage occurred at doses of 1250 and 1500 mcg/m² i.v.. Main toxicities were nausea and vomiting (requiring intravenous antiemetic treatment), myelosuppression and transient elevations in transaminases.

In addition, in a phase II study by i.v. route in breast cancer patients with liver metastases (previously untreated for the advanced disease), 1 complete response, 3 partial responses (one of them not confirmed four weeks apart) and 1 minor response were observed in liver lesions of six patients treated (at 1500 mcg/m² i.v.).

These findings suggest an interesting affinity of MMDX for liver lesions, even in tumor types resistant to conventional chemotherapy such as colorectal cancer and renal cancer.

Strong evidence of antitumor efficacy in liver is also supported by preclinical data. Activity of MMDX against liver metastases from M5076 murine reticulosarcoma is higher after oral administration as compared to the i.v. route, suggesting that a first pass effect may favour the efficacy in liver. In addition, MMDX administered orally is more effective on the liver metastases than on the solid primary in the same model. This specific effect on liver metastases is probably due to metabolite(s) produced by liver enzymes. This hypothesis is reinforced by several results showing MMDX being activated in vitro by liver microsomes to a highly cytotoxic product. This metabolic conversion is believed to occur also in humans.

The hints of activity observed in the current clinical experience, coupled with the activity of MMDX in mdr models and in liver metastasis models, raise the expectation of an improved clinical outcome for patients with hepatic neoplastic lesions.

It would be therefore desirable to establish drug delivery strategies to avoid the high i.v. dosages of MMDX presently believed to have an antitumor activity at the hepatic level and to improve the antitumor efficacy of MMDX against a primary liver cancer and liver metastases.

There is a need to achieve high MMDX concentration at the hepatic tumor site, while reducing systemic exposure and hence toxicity.

The present invention fulfills such a need by providing a new method for administration of MMDX to a patient suffering from a liver tumor wich reduces the MMDX amount without decreasing the MMDX's antitumor activity at the hepatic tumor site by directly injecting MMDX into the hepatic artery.

It is therefore a first object of the present invention the use of MMDX in the preparation of a medicament for the treatment of a human liver tumor which comprises intrahepatic administration of MMDX via the hepatic artery.

It is a still further object of the present invention a method for reducing MMDX systemic exposure of a patient suffering from a liver cancer which comprises the intrahepatic administration via the hepatic artery of a therapeutically effective amount of MMDX to said patient.

According to the present invention, a liver tumor can be a tumor primarily confined to the liver such as, e.g. an hepatocellular carcinoma or a cholangiocarcinoma, or a liver metastase.

MMDX is administered via the hepatic arthery, for example, as an infusion of from about 15 minutes to about 30 minutes every 4 weeks, or as a 5-10 minute bolus every 8 weeks, to adult patients with either a hepatic metastatic cancer, for example, patients with colorectal cancer who have progressed after receiving intravenous chemotheraphy or intrahepatic 5-fluorouracil or 5-fluorodeoxyuridine (FUDR) chemotheraphy, or patients with previously untreated primary liver carcinoma such as, for example, hepatocellular carcinoma or cholangiocarcinoma involving the liver.

In a more particular embodiment of the present invention, MMDX is administered to a patient in a dosage ranging from, e.g., about 100 mcg/m² to about 1000 mcg/m², preferably from about 100 mcg/m² to about 800 mcg/m², for example in a dosage of about 200 mcg/m².

In a still more particular embodiment of the present invention, the appropriate dose of MMDX, preferably previously dissolved in saline solution, is mixed with a suitable amount of an agent which remains selectively in a liver tumor after its injection through the hepatic arthery. Preferably, the amount of this agent, for example iodized oil (LIPIODOL®), may vary from about 3 ml to about 20 ml, depending on the tumor size.

LIPIODOL® is a lipid lymphographic agent which has been found to remain selectively in liver tumor after its injection through the hepatic arthery so it is particularly useful as a carrier of anticancer agents.

The following table illustrates the suitable LIPIODOL® volumes referred to tumor size.

**Table**

| **Tumor size (cm)** | **LIPIODOL® volume (ml)** |
|---|---|
| >= 2 | 3-5 |
| 2.1-6 | 5-8 |
| 6.1-11 | 9-14 |
| >11 | 15-20 |

The administration dosage of MMDX will vary depending upon the disease status of the patient.

The dosage regimen must therefore be tailored to the particular of the patient's conditions, response and associate treatments in a manner which is conventional for any therapy, and may need to be adjusted in response to changes in conditions and/or in light of other clinical conditions.

For example, for intrahepatic therapy, freeze-dried vials containg 500 mcg of MMDX is diluted with 5 ml of sterile saline for injection to obtain a MMDX concentration of 100 mcg/ml. The appropiate dose of MMDX to be given to the patient is optionally mixed with a suitable amount of LIPIODOL®.

The active drug can be administered directly into the lateral entry of an i.v. line inserted into the bung of an intrahepatic potacath lying beneath the upper anterior abdominal wall. The drug can administered, for example, over 30 minutes infusion in a volume of 100 ml of normal saline. Flushing of the device with 10-20 ml of saline can be done to assure that all the drug is given. Patients who do not have a portacath have a catheter inserted into the hepatic artery by a femoral Seldinger approach and the drug can be infused, for example, over 30 minutes infusion in a volume of 100 ml of normal saline. The catheter is inserted under local anesthesia and can then be removed from the groin, a pressure bandage applied and nursing observations continued overnight in hospital.

Object of this invention is also to provide a pharmaceutical composition comprising MMDX as the active substance, in association with a pharmaceutically acceptable agent, as defined above, which remains selectively in a liver tumor after its intrahepatic injection through the hepatic artery, and one or more pharmaceutically acceptable excipients and/or carriers. The pharmaceutical compositions are usually prepared following conventional methods and are administered in a pharmaceutically suitable form. For instance, solutions for intrahepatic injection or infusion may contain as a carrier, for example, sterile water or preferably, they may be in the form of sterile aqueous isotonic saline solutions.

The following Experimental Protocol illustrates the present invention.

### EXPERIMENTAL PROTOCOL

A single arm, multicentre, dose-finding Phase I study of MMDX (PNU 152243) administered as a brief infusion of 30 minutes every 4 weeks via the hepatic artery (IHA) to adult patients either with hepatic metastatic colorectal cancer who have progressed after receiving intravenous chemotherapy or intrahepatic 5-fluoruracil chemotherapy, or with previously untreated primary hepatocellular carcinoma or cholangiocarcinoma involving the liver was carried out. Their disease was confined to the liver, at the time of trial entry. Patients may have already had an intrahepatic portacath in situ or have the drug administered via the hepatic artery by a femoral Seldinger approach.

The primary goal of this study was the determination of Maximal Tolerated Dose (MTD) and Dose-Limiting Toxicities (DLTs)of MMDX when administered via the hepatic artery.

Antitumor activity was documented in this study.

The starting dose was 100 mcg/m²,corresponding to one third of the LD₁₀ in rats.

On a total of 23 registered patients, 18 received intra hepatic artery (IHA) administration of MMDX.

Doses tested ranged from 100 mcg/m² to 800 mcg/m².

Results are available on 13 patients: 3 patients (6 cycles) at 100 mcg/m², 3 patients (12 cycles) at 200mcg/m², 3 patients (4 cycles) at 400 mcg/m², 3 patients (10 cycles) at 600 mcg/m² and 1 patient (3 cycles) at 800 mcg/m².

### Hematological toxicity

Grade 1 leucopenia was observed in 1 patient at 100 mcg/m² (1 cycle), 1 patient (1 cycles) at 200 mcg/m² and 1 patient (1 cycle) at 400 mcg/m². AGC were however always normal. Grade 1-2 thrombocytopenia occured in 1 patient (3 cycles) at 200 mcg/m² and in 1 patient (1 cycle) at 600 mcg/m² and were considered tumor related (HCC patients). Max. grade 1 anemia was reported in three patients at 200, 600 and 800 mcg/m², respectively.

### Non-hematological toxicity

The most frequently observed adverse events, attributable to the study drug were nausea, vomiting and fatigue.

At 100 mcg/m²: mild vomiting was reported in one out of three patients (in 1 cycle); grade 1-2 fatigue was present in 2 patients (3 cycles).

At 200 mcg/m²: grade 2 nausea in 2 patients (2 cycles), grade 1-2 vomiting in 2 patients (3 cycles) and grade 2 fatigue in 1 patient (2 cycles) were reported.

At 400 mcg/m²: grade 2-3 nausea occurred in 2 patients (2 cycles) and grade 2 vomiting in one patient (1 cycle). Mild local pain at porth-a-cath site, and mild alopecia were reported in one patient (1 cycle).

At 600 mcg/m²: grade 1-2 nausea in 2 patients (2 cycles), grade 2 fatigue in 1 patient (2 cycles) and mild alopecia in 1 patient (2 cycles) were reported.

At 800 mcg/m²: grade 1 nausea, fatigue, mucositis and fever occurred in 1 patient (1 cycle each).

No other grade 3-4 events were reported.

Mild to moderate (grade 1-2) increase in transaminases (attributable to the study drug) was observed at 200 mcg/m² (2/3 patients), at 400 mcg/m² (2/3 patients; a third patient showed a grade 2-3 transaminase elevation probably due to the IHA technology applied), and at 600 mcg/m² in 2 out of 3 patients. A grade 3 transaminase elevation was reported in the patient treated at 800 mcg/m².

The maximum transaminases elevation appeared during the first week after treatment. Grade 2 bilirubinemia increases were observed starting from 100 mcg/m², but were not considered due to the study drug.

Other severe, non tumor related, laboratory abnormalities consisted of two grade 3 hyperglycemia observed in two patients treated at 200 mcg/m² (persisting from baseline in one diabetic patient and being post prandial in the other patient).

### Activity

Two objective tumor response were observed in liver at 200 mcg/m² in patients with HCC.

One patient had measurable liver disease at study entry followed by NMR (overall 17.75 cm²); after 2 IHA cycles, Partial Response (PR) was achieved (reduction by more than 86%); the PR was confirmed after the fourth IHA cycle and became Complete Response (CR) after the sixth IHA cycle; the patient went off therapy and, at the moment, he is relapse-free and in follow-up.

The second patient presented multiple liver lesions at baseline (the bigger one was 6 cm diameter, evaluated by Ctscan). Also in this case, PR was observed after 2 IHA cycles and confirmed after the third IHA cycle (the bigger lesion was decreased by 50% in diameter). Despite extrahepatic tumor progression (bone), the patient received another IHA treatment after which he was withdrawn from therapy. Two months later liver Ctscan was repeated and the previous findings on the bigger lesions were confirmed while the smallest lesions were slightly increased.

In a third HCC patient treated at 800 mcg/m², a minor response was reported after 3 cycles.

These activity data show that the MMDX chemotherapy through the hepatic artery is effective for patients with liver cancers at a MMDX dosage much lower than that employed by intravenous route, strongly reducincing the dangerous systemic exposure and hence toxicity of MMDX.

## Claims

1. Use of methoxymorpholino doxorubicin (MMDX) of formula 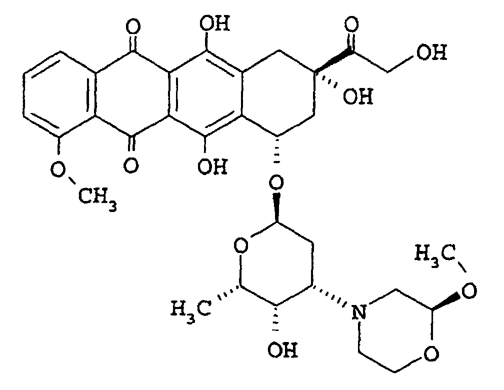 in the preparation of a medicament formulated for intrahepatic administration via the hepatic artery in the treatment of a human liver tumour.

2. Use according to claim 1. wherein the liver tumor is a tumor primarily confined to the liver.

3. Use according to claim 2. wherein the tumor primarily confined to the liver is a hepatocellular carcinoma (HCC) or a cholangiocarcinoma.

4. Use according to claim 1. wherein the tumor is a liver metastasis.

5. Use according to any one of the preceeding claims wherein MMDX is to be administered as an infusion of from about 15 minutes to about 30 minutes every 4 weeks.

6. Use according to anyone of claims 1. to 5. wherein MMDX is to be administered as a 5-10 minute bolus every 8 weeks.

7. Use according to any one of the preceeding claims wherein MMDX is to be administered with an agent which remains selectively in a liver tumor after its injection through the hepatic artery.

8. Use according to claim 7. wherein the agent is iodized oil.

9. Use according to anyone of the preceeding claims wherein MMDX is to be administered in a dose ranging from about 100 mcg/m² to about 1000 mcg/m².

10. Use according to claim 9. wherein MMDX is to be administered in a dose ranging form about 100 mcg/m² to about 800 mcg/m².

11. Use according to claim 10. wherein the dose is 200 mcg/m².

12. A pharmaceutical composition which comprises as an active principle MMDX and a pharmaceutically acceptable agent which is to remain selectively in a liver tumor after its injection through the hepatic artery.

13. A pharmaceutical composition according to claim 12. wherein the agent is iodized oil.

14. Use of a har aceutical composition according to claim 12. or 13. for the preparation of a medicament for the treatment of a liver tumor.

15. Use of a pharmaceutical composition which comprises as an active principle MMDX and a pharmaceutically acceptable agent which remains selectively in a liver tumor after its injection through the hepatic artery, for the preparation of a medicament formulated for intrahepatic administration in the treatment of a human liver tumor.

16. Use according to claim 15 wherein the agent is iodized oil.

## Patentansprüche

1. Verwendung von Methoxymorpholinodoxorubicin (MMDX) der Formel: 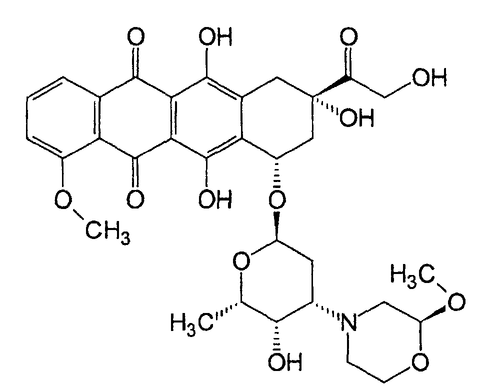 in der Herstellung eines Medikaments, das zur intrahepatischen Verabreichung über die Leberarterie in der Behandlung eines menschlichen Lebertumors formuliert ist.

2. Formulierung gemäss Anspruch 1, worin der Lebertumor ein primär auf die Leber beschränkter Tumor ist.

3. Verwendung gemäss Anspruch 2, worin der primär auf die Leber beschränkte Tumor ein Leberzellkarzinom (HCC) oder ein Cholangiokarzinom ist.

4. Verwendung gemäss Anspruch 1, worin der Tumor eine Lebermetastase ist.

5. Verwendung gemäss einem der vorhergehenden Ansprüche, worin MMDX als Infusion für ca. 15 bis ca. 30 Minuten alle 4 Wochen zu verabreichen ist.

6. Verwendung gemäss einem der Ansprüche 1 bis 5, worin MMDX als 5- bis 10-minütiger Bolus alle 8 Wochen zu verabreichen ist.

7. Verwendung gemäss einem der vorhergehenden Ansprüche, worin MMDX mit einem Mittel zu verabreichen ist, das selektiv in einem Lebertumor nach seiner Injektion durch die Leberarterie verbleibt.

8. Verwendung gemäss Anspruch 7, worin das Mittel iodiertes Öl ist.

9. Verwendung gemäss einem der vorhergehenden Ansprüche, worin MMDX in einer Dosis im Bereich von ca. 100 bis ca. 1.000 µg/m² zu verabreichen ist.

10. Verwendung gemäss Anspruch 9, worin MMDX in einer Dosis im Bereich von ca. 100 bis ca. 800 µg/m² zu verabreichen ist.

11. Verwendung gemäss Anspruch 10, worin die Dosis 200 µg/m² beträgt.

12. Pharmazeutische Zusammensetzung, die als Wirkstoff MMDX und ein pharmazeutisch akzeptables Mittel, das selektiv in einem Lebertumor nach seiner Injektion durch die Leberarterie verbleiben soll, umfasst.

13. Pharmazeutische Zusammensetzung gemäss Anspruch 12, worin das Mittel iodiertes Öl ist.

14. Verwendung einer pharmazeutischen Zusammensetzung gemäss Anspruch 12 oder 13 zur Herstellung eines Medikaments zur Behandlung eines Lebertumors.

15. Verwendung einer pharmazeutischen Zusammensetzung, die als Wirkstoff MMDX und ein pharmazeutisch akzeptables Mittel, das selektiv in einem Lebertumor nach seiner Injektion durch die Leberarterie verbleibt, umfasst, zur Herstellung eines Medikaments, das zur intrahepatischen Verabreichung in der Behandlung eines menschlichen Lebertumors formuliert ist.

16. Verwendung gemäss Anspruch 15, worin das Mittel iodiertes Öl ist.

## Revendications

1. Utilisation de la méthoxymorpholino doxorubicine (MMDX) de formule 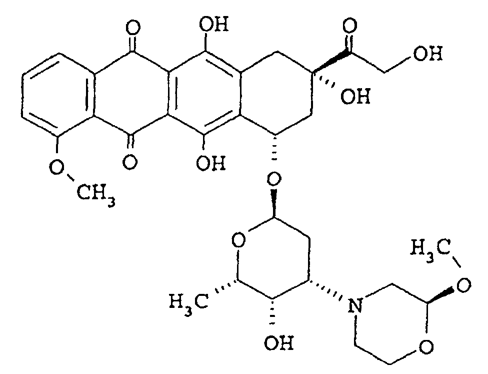 dans la préparation d'un médicament formulé pour une administration par voie intra-hépatique via l'artère hépatique dans le traitement d'une tumeur du foie humain.

2. Utilisation selon la revendication 1, dans laquelle la tumeur du foie est une tumeur essentiellement confinée au foie.

3. Utilisation selon la revendication 2, dans laquelle la tumeur essentiellement confinée au foie est un carcinome hépatocellulaire (CHC) ou un cholangiocar-cinome.

4. Utilisation selon la revendication 1, dans laquelle la tumeur est une métastase du foie.

5. Utilisation selon l'une quelconque des revendications précédentes, dans laquelle la MMDX doit être administrée sous forme d'une injection intraveineuse lente d'environ 15 minutes à environ 30 minutes toutes les 4 semaines.

6. Utilisation selon l'une quelconque des revendications 1 à 5, dans laquelle la MMDX doit être administrée sous forme d'un bolus de 5 à 10 minutes toutes les 8 semaines.

7. Utilisation selon l'une quelconque des revendications précédentes, dans laquelle la MMDX doit être administrée avec un agent qui reste sélectivement dans une tumeur du foie après son injection par l'artère hépatique.

8. Utilisation selon la revendication 7, dans laquelle l'agent est de l'huile iodée.

9. Utilisation selon l'une quelconque des revendications précédentes, dans laquelle la MMDX doit être administrée à une dose se situant dans la plage d'environ 100 mcg/m² à environ 1000 mcg/m².

10. Utilisation selon la revendication 9, dans laquelle la MMDX doit être administrée à une dose se situant dans la plage d'environ 100 mcg/m² à environ 800 mcg/m².

11. Utilisation selon la revendication 10, dans laquelle la dose est de 200 mcg/m².

12. Composition pharmaceutique qui comprend, en tant que principe actif, la MMDX et un agent pharmaceutiquement acceptable qui doit rester sélectivement dans une tumeur du foie après son injection par l'artère hépatique.

13. Composition pharmaceutique selon la revendication 12, dans laquelle l'agent est de l'huile iodée.

14. Utilisation d'une composition pharmaceutique selon la revendication 12 ou 13, pour la préparation d'un médicament pour le traitement d'une tumeur du foie.

15. Utilisation d'une composition pharmaceutique qui comprend, en tant que principe actif, la MMDX et un agent pharmaceutiquement acceptable qui doit rester sélectivement dans une tumeur du foie après son injection par l'artère hépatique, pour la préparation d'un médicament formulé pour une administration par voie intra-hépatique dans le traitement d'une tumeur du foie humain.

16. Utilisation selon la revendication 15, dans laquelle l'agent est de l'huile iodée.
